# EUROPÄISCHE PATENTANMELDUNG

(11) **EP 3 786 148 A1**
(43) Veröffentlichungstag der Anmeldung: **03.03.2021**
(21) Anmeldenummer: 19193981.8
(22) Anmeldetag: 28.08.2019
(51) Int. Cl.: C07C 67/39, C07C 67/58, C07C 69/54

(54) **VEREINFACHTE AUFARBEITUNG DES REAKTORAUSTRAGS EINER OXIDATIVEN VERESTERUNG**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein Verfahren zur Herstellung von Methylmethacrylat durch direkte oxidative Veresterung von Methacrolein. Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Insbesondere betrifft die vorliegende Erfindung eine optimierte Aufarbeitung des Reaktoraustrags der oxidativen Veresterung von Methacrolein, mittels dem nicht umgesetztes Methacrolein (MAL) sehr effizient recycliert werden kann. Außerdem kann dieser neue Prozess im Vergleich zu bekannten Varianten deutlich energie- und wassersparender betrieben werden.

## Beschreibung

### Gebiet der Erfindung

Die vorliegende Erfindung betrifft ein Verfahren zur Herstellung von Methylmethacrylat durch direkte oxidative Veresterung von Methacrolein. Methylmethacrylat wird in großen Mengen zur Herstellung von Polymeren und Copolymeren mit anderen polymerisierbaren Verbindungen eingesetzt. Weiterhin ist Methylmethacrylat ein wichtiger Baustein für diverse, auf Methacrylsäure (MAS) basierenden Spezialester, die durch Umesterung mit dem entsprechenden Alkohol hergestellt werden können. Hieraus ergibt sich ein großes Interesse an möglichst einfachen, wirtschaftlichen und umweltschonenden Herstellungsverfahren für diesen Ausgangsstoff.

Insbesondere betrifft die vorliegende Erfindung eine optimierte Aufarbeitung des Reaktoraustrags der oxidativen Veresterung von Methacrolein, mittels dem nicht umgesetztes Methacrolein (MAL) sehr effizient recycliert werden kann. Außerdem kann dieser neue Prozess im Vergleich zu bekannten Varianten deutlich energie- und wassersparender betrieben werden.

### Stand der Technik

Methylmethacrylat (MMA) wird heute mittels diverser Verfahren, die von C₂- C₃- oder C₄-Bausteinen ausgehen, hergestellt. In einem dieser Verfahren wird MMA durch Oxidation von Isobutylen oder tert-Butanol mit Luftsauerstoff in der Gasphase am heterogenen Kontakt zu Methacrolein und anschließender oxidativer Veresterungsreaktion von Methacrolein unter Verwendung von Methanol erhalten. Dieses von ASAHI entwickelte Verfahren ist unter anderem in den Druckschriften US 5,969,178 und US 7,012,039 beschrieben. Nachteil dieses Verfahrens ist insbesondere ein sehr hoher Energiebedarf. In einer Weiterentwicklung des Verfahrens wird das Methacrolein in der ersten Stufe aus Propanal und Formaldehyd gewonnen. Ein solches Verfahren ist in WO 2014/170223 beschrieben.

Das erfindungsgemäße Verfahren umfasst auch die Herstellung von Methacrolein, bevorzugt in einem kontinuierlichen Prozess und mit der besonderen Maßgabe, dass eine Reinheit und Qualität von Methacrolein erzeugt wird, die für den nachfolgenden Umsetzungsschritt akzeptabel ist, der direkten oxidativen Veresterung von Methacrolein zu MMA. Nach Stand der Technik zur Herstellung von Methacrolein kommen verschiedene Verfahren und Ausführungsformen in Frage, die es erlauben das vorliegende erfindungsgemäße Verfahren durchzuführen.

Bei der Herstellung von Methacrolein nach dem sogenannten C2-Verfahren wird aus Formalin und Propionaldehyd in Gegenwart eines sekundären Amins und einer Säure, zumeist einer organischen Säure, das Zielprodukt erhalten. Die Umsetzung erfolgt dabei über eine Mannich- ähnliche Reaktion mit anschließender Freisetzung des katalytischen sekundären Amins.

Das erfindungsgemäße Verfahren umfasst auch die Herstellung von Methacrolein, bevorzugt in einem kontinuierlichen Prozess und mit der besonderen Maßgabe, dass eine Reinheit und Qualität von Methacrolein erzeugt wird, die für den nachfolgenden Umsetzungsschritt, der direkten oxidativen Veresterung von Methacrolein zu MMA, akzeptabel ist. Nach Stand der Technik zur Herstellung von Methacrolein kommen verschiedene Verfahren und Ausführungsformen in Frage, die es erlauben das vorliegende erfindungsgemäße Verfahren durchzuführen.

Grundsätzlich erfolgt nach diesem Mechanismus eine Kondensation des Formalins mit Propanal, wobei Wasser als Kondensationsprodukt formal erzeugt wird. Das so synthetisierte Methacrolein (MAL) kann dann in einem nachfolgenden Schritt durch Oxidation in der Gasphase zu Methacrylsäure oder durch oxidative Veresterung zu Methylmethacrylat umgesetzt werden. Solche Verfahren zur Herstellung von Methacrolein, sind unter anderem in den Druckschriften US 7,141,702, US 4,408,079, JP 3069420, JP 4173757, EP 0 317 909 und US 2,848,499 beschrieben.

Die zur Herstellung von Methacrolein geeigneten, auf einer Mannich-Reaktion basierenden Verfahren sind dem Fachmann im Allgemeinen bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Auch Ausführungsformen zur Erzeugung von Methacrolein aus Propanal und Formalin sind bekannt die nicht in Gegenwart von Aminen als Katalysatoren arbeiten, z.B. die Herstellung in der Gasphase in der Gegenwart von Festbettkatalysatoren, wie jüngst von Eastman publiziert.

Für eine wirtschaftliche Nutzung dieses Verfahrens ist eine hohe Ausbeute und ein geringer spezifischer Energiebedarf zu erreichen. Nach der Lehre der EP 0 194 620 ist ein kleiner Gehalt an dimeren Methacrolein (DIMAL) im Produkt, bevorzugt kleiner 0,2 Gew%, bzw. ein Propionaldehydgehalt (Propanal) von kleiner 1 Gew% anzustreben, um eine nachhaltige Schädigung des Oxidationskatalysators einer optional nachfolgenden heterogenen Gasphasenkatalyse zu vermeiden. In DE 3213681 wird ein Verfahren zur Herstellung von MAL beschrieben, welches insbesondere dadurch charakterisiert ist, dass die Umsetzung bei einer Temperatur von größer 150 °C bei einer Reaktionszeit von höchstens 25 min in Gegenwart von sekundären Aminen und optional von Säuren durchgeführt wird. Bestenfalls wird dabei Propionaldehyd mit Formalin bei Temperaturen von 162 bis 205 °C sowie einer Verweilzeit von 6 Sekunden umgesetzt.

In einer weiteren Ausführungsform der DE 3213681 wird der Reaktor bei einer Eingangstemperatur von 161 °C betrieben und durch die stark exotherme Reaktion steigt die Temperatur auf bis zu 184 °C an. Die Verweilzeit beträgt ca. 6,9 sec. Der Wassergehalt im Feed zur Reaktion beträgt ca. 50 Gew%. Der auf das Wasser bezogene Amin-Gehalt beträgt 1,8 Gew%. So wird beispielsweise mit einer solchen Verfahrensvariante bei einer Ausbeute von 98,1 % ein DIMAL-Gehalt von 0,29 Gew% beobachtet.

US 4,408,079 beschreibt ein Verfahren zur Herstellung von MAL, bei dem die Umsetzung von Propionaldehyd mit Formalin bei einem Molverhältnis von 0,9 bis 1,5 zu 1 , einem pH-Wert zwischen 2,5 und 7 und Temperaturen von 0 °C bis 150 °C in Gegenwart von einem sekundären Amin, in einer Konzentration von 0,025 bis 0,75 bzw. von 0,05 bis 1,5 mol, und organischen Säuren in einer Konzentration von 0,05 bis 1,5 mol jeweils bezogen auf 1 mol Propionaldehyd durchgeführt wird. Im Vergleich zu der Lehre der DE 3213681 ist der gewählte Temperaturbereich somit deutlich tiefer. Dabei wird gemäß US 4,408,079 die Reaktion in einer Rührkesselkaskade von zwei bis drei Reaktoren kontinuierlich bei sehr langen Verweilzeiten der Reaktanden von 10 bis 90 min durchgeführt. Es werden mit dieser Ausführungsform des Verfahrens relativ niedrige Ausbeuten von 91 bis 96 % erzielt.

Zusammenfassend lässt sich sagen, dass zwar alle diese Verfahren sich sehr wesentlich in Verweilzeit, Katalysatormenge und dem verwendeten Reaktortypen unterscheiden, allerdings bezgl. Ausbeute und Methacrolein Qualität grundsätzlich geeignet sind, um für eine nachfolgende Umsetzung gemäß einer direkten oxidativen Veresterung des Methacroleins zu MMA in der Flüssigphase eingesetzt zu werden. Allen Verfahren ist gemeinsam, dass die Herstellung von Methacrolein durch Kondensation von wässrigen Formalin Lösungen mit Propanal in einem oder mehreren Reaktoren durchgeführt werden (Die Reaktoren können parallel oder kaskaden-artig miteinander in Verbindung stehen) und dass Methacrolein so aufgearbeitet wird, dass eine weitgehende Abtrennung von einer wässrigen Phase erfolgt, die größtenteils das Prozesswasser sowie den Wasseranteil, der mit Formaldehyd in die Reaktion gelangt, enthält. Die Abtrennung dient dem weiteren Zweck, dass das erzeugte reine Methacrolein weitestgehend von Katalysatorspuren befreit ist, da die Möglichkeit besteht, dass Katalysatorreste die Performance der nachfolgenden DOE Katalysatoren negativ beeinflusst.

Der Herstellung von MMA aus Methacrolein in der sogenannten direkten oxidativen Veresterung in der Flüssigphase mit Luft und Methanol als Reaktanten kommt für die vorliegende Erfindung besondere Bedeutung zu.

In US 5,969,178 ist ein solches Verfahren zur oxidativen Umsetzung von Isobuten oder tert-Butanol zu Methacrolein und die folgende oxidative Veresterung zu MMA beschrieben. In dieser zweiten Stufe wird eine flüssige, im Wassergehalt reduzierte Mischung aus Methacrolein und Methanol mit molekularem Sauerstoff und einem Palladiumkatalysator umgesetzt, wobei dieser zumeist auf einem Träger als Palladium- Blei-Katalysator vorliegt. Aus dem Rohprodukt der oxidativen Veresterung wird darauf in einer ersten Destillationsstufe ein Gemisch aus Methacrolein und Methanol unterhalb des Kopfes der Kolonne abgetrennt, während über Kopf leichtsiedende Bestandteile entfernt werden. Der MMA-haltige Sumpf wird darauf in eine zweite Destillationsstufe geführt, in der über Kopf ein Azeotrop aus Methanol und gesättigten Kohlenwasserstoffen abgetrennt wird. Der Sumpf, enthaltend das Roh-MMA wird einer weiteren Aufarbeitung zugeführt, während aus der über Kopf gewonnen Fraktion mittels einem Phasentrenner und einer dritten Destillationskolonne Methanol isoliert und zurück in den Reaktor geführt wird. Dabei ist zu berücksichtigen, dass das Methanol aufgrund des gebildeten Azeotrops relativ viel Wasser enthalten kann und damit einer Entwässerung zugeführt werden muss. Als Alternative zu diesem Verfahren offenbart die US 5,969,178 die Aufarbeitung in nur einer Kolonne, wobei sich bei dieser der Zulauf zwingend oberhalb des Sumpfes befindet. Über Kopf werden dieser Kolonne leichtsiedende Bestandteile aus dem Reaktoraustrag entfernt. Im Sumpf verbleibt eine Mischung aus Roh-MMA und Wasser, welche einer weiteren Aufarbeitung zuzuführen ist. Über einen Seitenstrom, dessen genaue Lage zunächst bestimmt werden muss und die durch Hinzufügen verschiedener Siedeböden eingestellt werden kann, wird der Kolonne schließlich eine Mischung aus Methacrolein und Methanol, gedacht zur Zurückführung in den Reaktor, entnommen. US 5,969,178 weist dabei selbst darauf hin, dass ein solches Verfahren aufgrund diverser Azeotrope schwierig durchzuführen ist. Darüber hinaus spielt dabei insbesondere Methacrylsäure, die immer als Nebenprodukt vorliegt eine große Rolle. Nach diesem Verfahren, auch wenn die US 5,969,178 darüber schweigt, würde die Methacrylsäure derart abgetrennt werden, dass sie in einer der Entsorgung zuzuführenden Phase verbleibt und sich eine Isolierung nur bedingt lohnen würde. Damit jedoch sinkt die Gesamtausbeute an methacrylischen Produkten dieses Verfahrens.

In US 7,012,039 wird eine etwas abweichende Aufarbeitung des Reaktoraustrags der oxidativen Veresterung offenbart. Hier wird in einer ersten Destillationsstufe über Siebböden Methacrolein über Kopf abdestilliert und die wässrige, MMA haltige Mischung aus dem Sumpf in einen Phasenseparator geleitet. In diesem wird die Mischung durch Zugabe von Schwefelsäure auf einen pH-Wert von ca. 2 eingestellt. Die Trennung des schwefelsauren Wassers von der organischen bzw. Öl-Phase erfolgt dann mittels Zentrifugierens. Diese Ölphase wird in einer weiteren Destillation in hochsiedende Bestandteile und eine MMA haltige Phase, welche über Kopf abgezogen wird, getrennt. Die MMA haltige Phase wird danach in einer dritten Destillation von niedrigsiedenden Bestandteilen getrennt. Darauf folgt noch eine vierte Destillation zur endgültigen Reinigung.
Problematisch an diesem Verfahren ist die Schwefelsäure, die in großen Mengen zugegeben werden muss und in Teilen der Anlage korrosiv wirken kann. Entsprechend müssen diese Teile, wie insbesondere der Phasenseparator oder auch die zweite Destillationskolonne aus dafür geeigneten Materialien gefertigt sein. Weiterhin schweigt auch die US 7,012,039 über den Umgang mit der gleichsam anfallenden Methacrylsäure oder dem im Produkt verbleibenden restlichen Methanol. Es ist jedoch zu vermuten, dass die erstgenannte in den Destillationsstufen mit abgetrennt wird, während das Methanol nur teilweise mit dem Methacrolein gewonnen und zurückgeführt werden kann, während der Rest wahrscheinlich in der dritten Destillationsstufe verloren geht.

WO 2014/170223 beschreibt ein ähnliches Verfahren wie US 7,012,039. Es besteht nur der Unterschied, dass in der eigentlichen Reaktion mittels Zugabe einer methanolischen Natriumhydroxidlösung in einer Kreislaufführung der pH-Wert eingestellt wird. Dies dient unter anderem dazu, den Katalysator zu schonen. Weiterhin ist die Abtrennung der wässrigen Phase in der Phasenseparation aufgrund des Salzgehalts einfacher. Es führt jedoch auch dazu, dass die entstehende Methacrylsäure als Natriumsalz vorliegt und später mit der wässrigen Phase abgetrennt und verworfen wird. Bei der Variante einer Schwefelsäurezugabe in der Phasenseparation wird die freie Säure zwar wiedergewonnen. Dafür fällt jedoch Natrium(hydrogen)sulfat an, welches bei der Entsorgung zu anderen Problemen führen kann.

EP 3 350 153 beschreibt ein Verfahren, bei dem aus dem Reaktoraustrag einer oxidativen Veresterung von Methacrolein nicht umgesetzter Alkohol destillativ abgetrennt und in den Reaktor recycliert wird.

Allen diesen Verfahren und Aufarbeitungsvarianten ist gemeinsam, dass der Umsatz der DOE Reaktion nicht vollständig ist. Somit besteht die Notwendigkeit, nicht umgesetztes Methacrolein und überschüssigen Alkohol, insbesondere im Falle der MMA-Herstellung überschüssiges Methanol in einer Kolonne, die direkt dem Reaktor nachgeschaltet ist, zu recyclieren. Diese Kolonne hat einen immensen Dampf- und Kühlwasserverbrauch und verbraucht somit nicht nur große Mengen Wasser, sondern auch Energie.

Zusammengefasst sind die folgenden Aspekte der Verfahren gemäß Stand der Technik vor allem in der Kombination miteinander verbesserungswürdig:
- Möglichst hohe Ausbeute
- Möglichst hohen Grad des Recyclings des nicht umgesetzten Methacroleins und Alkohols (Methanol)
- Möglichst geringer Gehalt, bzw. gegenüber dem Stand der Technik reduzierte Gehalt an Isobuttersäurealkylester, insbesondere Isobuttersäuremethylester im produzierten Alkylmethacrylat, insbesondere MMA
- Möglichst geringer Wasser- und Energieverbrauch, sowie saubere Entsorgungsströme bzw. Abgase

### Aufgabe

In Anbetracht des Standes der Technik ist es daher Aufgabe der vorliegenden Erfindung, ein technisch verbessertes Verfahren zur oxidativen Veresterung von Methacrolein zur Verfügung zu stellen, das nicht mit den Nachteilen herkömmlicher Verfahren behaftet ist.

Insbesondere war es Aufgabe der vorliegenden Erfindung, eine Verbesserung der Aufarbeitung des Rohproduktes einer oxidativen Veresterung von Methacrolein mit Methanol zu MMA bereitzustellen und damit die Gesamtausbeute eines solchen Prozesses gegenüber dem Stand der Technik zu verbessern.

Darüber hinaus war es Aufgabe, möglichst viele nicht umgesetzte Edukte oder im Prozess gebildete Neben- oder Zwischenprodukte, insbesondere Methanol, Methacrolein und Wasser, in möglichst hohem Maße zu recyclieren und im Prozess zu den Zielprodukten umzusetzen.

Darüber hinaus lag der vorliegenden Erfindung die Aufgabe zugrunde, die Isolierung von nicht umgesetzten Edukten, insbesondere Methacrolein und der Alkohol, aus dem Reaktoraustrag der oxidativen Veresterung, derart zu verbessern, dass diese mit weniger benötigter Energie und weniger Kühlwasser durchgeführt werden kann.

Insbesondere bestand auch die Aufgabe, ein Verfahren zur Verfügung zu stellen, das mit einem möglichst geringen Entsorgungsaufwand, insbesondere durch ein reduziertes Anfallen organischer Bestandteile und Säuren im Abfallstrom, betrieben werden kann.

Weiterhin soll das Verfahren gegenüber dem Stand der Technik kostengünstig, insbesondere in Bezug auf die bei der Konstruktion der Anlage zu verwendenden Materialien sein.

### Lösung

Gelöst werden die Aufgaben durch ein Verfahren zur Herstellung von MMA, bei dem in einer ersten Reaktionsstufe in einem Reaktor I Methacrolein hergestellt und dieses in einer zweiten Reaktionsstufe in einem Reaktor II oxidativ mit einem Alkohol zu einem Alkylmethacrylat verestert wird. Dabei ist das erfindungsgemäße Verfahren dadurch gekennzeichnet, dass zunächst der Reaktoraustrag aus Reaktor II erst in eine erste Phase, enthaltend mehr als 80 Gew% des im Reaktoraustrag vorliegenden Alkohols und eine zweite Phase, enthaltend jeweils mehr als 90 Gew% des im Reaktoraustrag vorliegenden Alkylmethacrylats und Methacroleins (MAL) getrennt wird. Daraufhin wird diese zweite Phase erst in eine dritte Phase, enthaltend schwersiedende Komponenten und eine vierte Phase, enthaltend jeweils mehr als 90 Gew% des im Reaktoraustrag vorliegenden Alkylmethacrylats und Methacroleins (MAL) getrennt. Anschließend wird diese vierte Phase in einer Destillationskolonne der überwiegende Teil des Alkylmethacrylats vom überwiegenden Teil des MAL getrennt.

Eine Vielzahl an möglichen Methoden und Verfahren kann zur Herstellung von Methacrolein als Vorstufe der DOE eingesetzt werden, unter anderem verschiedene im Stand der Technik dargestellten Verfahren, die von Propionaldehyd und Formalin ausgehen, als auch etablierte Verfahren ausgehend von Isobuten oder Isobuten Vorstufen wie MTBE und tert-Butanol, die in der Gasphase als Luftoxidation in Rohrbündelreaktoren an geeigneten heterogenen Kontakten durchgeführt werden.

Insbesondere Bedeutung hat dieses neuartige Verfahren für eine Durchführung, bei der es sich bei dem Alkohol um Methanol und bei dem Alkylmethacrylat um MMA handelt.

Als besonders vorteilhaft und energiesparend hat es sich erwiesen, wenn die vierte Phase erst in einem Phasentrenner von mindestens 90 Gew% des in der vierten Phase vorliegenden Wassers getrennt wird, bevor in einer Destillationskolonne der überwiegende Teil des Alkylmethacrylats vom überwiegenden Teil des MAL getrennt wird.

Als besonders bevorzugt hat es sich erwiesen, die vierte Phase oder die organische Phase der vierten Phase nach einer Trennung von einer wässrigen Phase in einem Phasentrenner in einer Destillationskolonne in eine leichtsiedende Fraktion, enthaltend mindestens 60 Gew% des MAL des Reaktoraustrags und eine schwersiedende Phase, enthaltend mindestens 90 Gew% des MMA des Reaktoraustrags zu trennen.

Dazu alternativ ist eine Variante, bei der die leichtsiedende, MAL enthaltende Fraktion, die aus der vierten Phase gewonnen wurde, in einer weiteren Kolonne von leichtsiedenden Bestandteilen getrennt wird. Die dabei gewonnene schwersiedende Fraktion dieser Kolonne, enthaltend mindestens 60 Gew%, bevorzugt mindestens 70 Gew% des MAL des Reaktoraustrags wird darauf in den Reaktor II oder einen dem Reaktor II vorgeschalteten Mischer oder Wärmetauscher geleitet.

Ganz besonders bevorzugt ist es dabei, die MAL enthaltene Fraktion, die aus der vierten Phase gewonnen wurde, erst in einem Phasentrenner von einer wässrigen Phase zu trennen, bevor die destillative Trennung von leichtsiedenden Bestandteilen und die Weiterleitung der MAL-Fraktion in oder vor Reaktor II erfolgt. Dieses Vorgehen ist insbesondere bevorzugt, wenn dieser zweite Phasentrenner und der erste, der destillativen Trennung der vierten Phase vorgeschaltete Phasentrenner beide eingesetzt werden.

Die anfallenden wässrigen Phasen der Phasentrenner, die im Falle mehrerer solcher Funktionsteile miteinander zusammengeführt werden können, können vor der endgültigen Entsorgung einer Membrantrennstufe zugeführt werden. Die dabei zurückbleibende, eher organische Phase kann optional im Weiteren derart destilliert werden, dass je nach Fahrweise vorliegendes MAL, Methanol und/oder MMA isoliert und zurückgeführt bzw. der MMA-Endreinigung zugeführt werden kann. Je nach konkreter Zusammensetzung dieser Phase kann diese auch direkt in die erste Destillation nach dem Austrag aus Reaktor II zur Isolierung von Methanol geleitet werden. Sollte überwiegend MAL zurückgewonnen werden, kann die Phase auch in einen der Phasentrenner, die Destillationskolonne zur endgültigen MAL-gewinnung oder - bevorzugt - in die Destillationskolonne zur Trennung der zweiten Phase in die dritte und die vierte Phase geleitet werden.

In Bezug auf die Trennung des Reaktoraustrags in die erste und die zweite Phase hat es sich als vorteilhaft erwiesen, wenn der Reaktoraustrag aus Reaktor II erst in einem Konverter mit einer starken Säure und optional zusätzlichem Wasser versetzt wird, bevor eine extraktive Trennung der beiden Phasen voneinander erfolgt. Anschließend wird die erste Phase, nur sehr wenig MAL enthaltend, in einer weiteren Destillationskolonne in eine überwiegend Alkohol enthaltende Phase und eine schwersiedende Phase, die entsorgt wird, getrennt. Die den Alkohol enthaltende Phase wird bevorzugt in den Reaktor II recycliert.

Besonders bevorzugt wird bei diesem Vorgehen so viel der starken Säure zugegeben, dass der pH-Wert bei der Extraktion immer kleiner als 7, bevorzugt kleiner/gleich 5, aber immer größer/gleich 1 ist. Die bevorzugte Säuremenge entspricht einer Zugabemenge bzw. Konzentration, die gewährleistet, dass die Materialien der Apparate nicht zu teuer und hochlegiert ausfallen aber auch die gewünschten chemischen Reaktionen ausreichend schnell und vollständig ablaufen. Ein solcher besonders bevorzugter pH Bereich beträgt zwischen pH 1,5 bis pH 4. Üblicherweise stellt ein pH zwischen 2 und 3 einen optimalen Kompromiss aller gewünschten Merkmale dar.

Durch die erfindungsgemäßen Maßnahmen, dass man nach der Abtrennung des Methanols aus dem Reaktoraustrag erst Schwersieder entfernt, bevor das Produkt in Form des Alkylmethacrylats und das Methacrolein voneinander getrennt werden, erzielt man diverse Vorteile. Durch die besonders bevorzugte Ausführung vor der Destillation zur Abtrennung des MALs eine Phasentrennung zur Entfernung von Wasser, werden diese Vorteile zusätzlich noch verstärkt. Bei den einzeln wie besonders auch in Kombination zueinander überraschend erzielten Vorteilen des erfindungsgemäßen Verfahrens handelt es sich insbesondere um Folgende:
Mit der insbesondere effektiver gestalteten Rückgewinnung des MAL wird die Gesamtausbeute eines solchen Prozesses gegenüber dem Stand der Technik überraschend erhöht. Insbesondere gelang es, im Reaktor zunächst nicht umgesetzte Edukte besonders effizient zu isolieren und damit einer Rückführung in den Reaktor in hohem Maße zu realisieren. Dies betrifft neben Methanol und Methacrolein und die Isolierung des Wassers, welches in der Aufarbeitung teilweise wiedereingesetzt werden kann, was wiederum die Abwassermengen des Gesamtprozesses reduziert.

Auch gelang es mit dem erfindungsgemäßen Verfahren sehr überraschend, den Gehalt an Isobuttersäurealkylester, insbesondere Isobuttersäuremethylester im produzierten Alkylmethacrylat, insbesondere MMA gegenüber dem Stand der Technik zu reduzieren. Dies kann eventuell auf die geringere thermische Belastung in der Aufarbeitung des Reaktoraustrags bis zur MAL-Abtrennung erklärt werden.

Weiterhin war es überraschend, dass mit dem erfindungsgemäßen Verfahren, insbesondere verstärkt in der Variante eines zwischengeschalteten Phasentrenners vor der MAL-Abtrennung, die Aufgabe, die Isolierung von nicht umgesetzten Edukten, insbesondere von Methacrolein und des Alkohols aus dem Reaktoraustrag der oxidativen Veresterung, derart zu verbessern, dass diese mit weniger benötigter Energie und weniger Kühlwasser durchgeführt werden kann. Darüber hinaus ist überraschend auch die Gesamtmenge der - sowohl wässrigen als auch organischen - Abfallströme gegenüber dem Stand der Technik überraschend reduziert.

Alternativ zum Verfahren wie im experimentellen Teil detailliert beschrieben, sind weitere Verschaltungen und Abfolgen der diversen Destillationskolonnen denkbar. Beispielsweise kann die organische Phase der Extraktion ("zweite Phase"), die vor allem aus MMA und Methacrolein besteht, in einer vorteilhaften Variante zunächst in der Leichtsiederkolonne aufbereitet werden, wobei MMA im Sumpf der Leichtsiederkolonne anfällt und anschließend der Aufarbeitung in der Hochsiederkolonne zugeführt wird. Hierbei fällt damit MMA in Monomer Qualität als Destillat der Hochsiederkolonne an, im Vergleich zu anderen denkbaren Verschaltungen entfällt nach dieser Variante die MMA Reinkolonne.

### Experimenteller Teil:

### 1. Kontinuierliche Durchführung der Herstellung von Methacrolein durch Kondensation von Formalin ("FA") mit Propanal

### Herstellung des Ausgangsstoffes für die DOE (siehe 2.a.)

Methacrolein kann gemäß DE3213681A1 kontinuierlich hergestellt werden. Reaktionsbedingungen und Ausbeuten sind für die Beispiele 1.1. und 1.2. gemäß DE321368A1 in Tabelle 1 dargestellt. Bei diesen Beispielen wird Methacrolein bei relativ hohen Temperaturen kontinuierlich hergestellt.

Beispiele 1.3. bis 1.19. gemäß EP2998284A1 beschreiben ebenfalls ein kontinuierliches Verfahren allerdings im Vergleich zu Beispiel 1.1. und 1.2. bei deutlich niedrigeren Temperaturen. Bei diesen Beispielen werden Formalinlösungen unterschiedlicher Konzentration (37 Gew% oder 55 Gew%) und Propionaldehyd mittels eines statischen Mischers vermischt (im Weiteren als Aldehydlösung bezeichnet) und anschließend in einem ölbeheizten Wärmetauscher auf die gewünschte Temperatur (siehe Tab.1) erwärmt. Ein Recyclestrom aus dem Sumpf der Produktkolonne, die an den Rohrreaktor anschließt, wird mit Essigsäure und Dimethylamin (als 40 %ige Lösung in Wasser) vermischt und ebenfalls auf die gewünschte Temperatur vorgewärmt. Die vorgewärmte Aldehydlösung und die vorgewärmte Katalysatorlösung werden in einem weiteren statischen Mischer vermischt. Diese Eduktmischung wird dann einem mittels Öl temperierten Rohreaktor zugeführt. Üblicherweise wird die Reaktion bei Drücken von ca. 35 bis 40 bar durchgeführt, um Ausgasung von Komponenten in der Reaktionszone vorzubeugen. Die Produktmischung am Ablauf des Rohrreaktors wird über ein Ventil entspannt und gelangt in die Produktkolonne zur Destillation. Am Kopf dieser Kolonne wird nach Kondensation und Phasentrennung ein zweiphasiges Gemisch aus Methacrolein und einer wässrigen Phase erhalten. Die wässrige Phase wird in die Kolonne als Rücklauf zurückgeführt. Die organische Phase gelangt in die Produktvorlage. Am Sumpf der Kolonne wird ein Teilstrom als Recycle in die Reaktion zurückgeführt. Ein weiterer Teilstrom wird als wässriges Produkt in eine weitere Produktvorlage abgeführt. Die Umsätze in allen Beispielen liegen über 99,3 % und die erzielten DIMAL - Gehalte liegen zwischen 0,18 und etwa 1,4 Gew% im isolierten Methacrolein.

| **Tabelle 1** | PA:FO | DMA:PA | ACOH:DMA | Recycle | DMA:PA | H₂O | DMA/H2O | VWZ | T_{ÖL} | Tₑᵢₙ | Tₘₐₓ | Tₐᵤₛ | Umsatz PA | Selkt. MAL | c DIMAL |
|---|---|---|---|---|---|---|---|---|---|---|---|---|---|---|---|
| | Frischfeed | | | | Reaktoreingang | | | | | | | | | | |
| | mol/mol | mol% | mol/mol | % | mol% | % | % | sec | °C | °C | | °C | % | % | % |
| DE3213681A1 Bsp 1.1. | 1 | 3,7 | 1,08 | - | - | 50 | 1,8 | 6,9 | | 161 | 184 | - | 99,5 | 98,1 | 0,49 |
| DE3213681A1 Bsp 1.2. | 1 | 3,6 | 1,14 | - | - | 40 | 2,5 | 6 | | 162 | 205 | - | > 99,4 | 97,2 | <1 |
| Beispiel 1.3. | 0,99 | 2,50 | 1,09 | 70,5 | 7,8 | 55,6 | 2,74 | 9,30 | 139,5 | 122,5 | 152,6 | 152,2 | 99,37 | 98,75 | 0,18 |
| Beispiel 1.4. | 0,99 | 2,51 | 1,09 | 71,0 | 7,8 | 56,1 | 2,74 | 9,26 | 139,1 | 122,5 | 152,3 | 152,0 | 99,30 | 98,85 | 0,18 |
| Beispiel 1.5. | 0,98 | 2,61 | 1,09 | 71,2 | 8,2 | 54,9 | 2,82 | 9,41 | 139,9 | 122,1 | 152,3 | 152,2 | 99,35 | 98,67 | 0,18 |
| Beispiel 1.6. | 0,96 | 2,51 | 1,09 | 70,1 | 7,7 | 56,5 | 2,71 | 9,21 | 139,1 | 122,8 | 153,0 | 153,0 | 99,46 | 98,33 | 0,18 |
| Beispiel 1.7. | 0,99 | 2,51 | 1,09 | 70,5 | 7,8 | 55,7 | 2,75 | 9,26 | 143,9 | 129,9 | 160,2 | 155,5 | 99,75 | 98,19 | 0,34 |
| Beispiel 1.8. | 0,99 | 2,51 | 1,09 | 70,4 | 7,8 | 55,6 | 2,75 | 9,30 | 144,2 | 127,3 | 157,7 | 154,7 | 99,65 | 98,47 | 0,27 |
| Beispiel 1.9. | 0,98 | 2,50 | 1,09 | 70,5 | 7,7 | 56,0 | 2,72 | 9,22 | 139,0 | 122,5 | 156,3 | 154,9 | 99,57 | 98,62 | 0,22 |
| Beispiel 1.10. | 0,99 | 2,51 | 1,09 | 70,5 | 7,8 | 55,6 | 2,74 | 9,26 | 159,8 | 142,1 | 173,0 | 169,1 | 99,67 | 98,03 | 0,49 |
| Beispiel 1.11. | 0,99 | 2,52 | 1,08 | 70,4 | 7,8 | 55,6 | 2,76 | 9,26 | 146,4 | 133,8 | 165,4 | 159,7 | 99,77 | 98,34 | 0,45 |
| Beispiel 1.12. | 0,99 | 2,50 | 1,09 | 48,8 | 4,7 | 56,4 | 1,76 | 9,23 | 159,5 | 141,0 | 172,3 | 169,6 | 99,65 | 98,02 | 0,49 |
| Beispiel 1.13. | 1,01 | 2,49 | 1,10 | 52,8 | 5,2 | 58,4 | 1,73 | 11,26 | 164,1 | 155,7 | 179,6 | 172,7 | 99,81 | 98,51 | 1,35 |
| Beispiel 1.14. | 1,00 | 2,52 | 1,09 | 53,5 | 5,2 | 58,7 | 1,73 | 11,23 | 164,0 | 150,3 | 177,5 | 168,7 | 99,83 | 98,40 | 1,13 |
| Beispiel 1.15. | 1,00 | 2,50 | 1,11 | 49,3 | 4,8 | 68,5 | 1,75 | 9,33 | 158,2 | 147,2 | 173,0 | 164,4 | 99,81 | 97,91 | 0,92 |
| Beispiel 1.16. | 1,00 | 4,83 | 1,10 | 16,5 | 5,7 | 41,8 | 3,78 | 12,27 | 149,1 | 150,5 | 179,8 | 167,8 | 99,32 | 96,44 | 0,93 |
| Beispiel 1.17. | 0,99 | 4,81 | 1,09 | 16,7 | 5,7 | 42,3 | 3,73 | 12,21 | 148,4 | 142,2 | 172,2 | 158,6 | 99,17 | 98,05 | 0,65 |
| Beispiel 1.18. | 0,99 | 4,01 | 1,10 | 16,6 | 4,8 | 41,7 | 3,11 | 12,32 | 153,6 | 141,5 | 171,9 | 159,2 | 99,66 | 98,58 | 0,68 |
| Beispiel 1.19. | 1,00 | 2,50 | 1,10 | 65,3 | 6,7 | 83,9 | 1,66 | 7,66 | 158,8 | 154,2 | 171,7 | 171,7 | 99,58 | 98,18 | 0,94 |
| Beispiel 1.20. | 1,01 | 2,50 | 1,10 | 65,0 | 6,5 | 64,8 | 1,63 | 7,68 | 158,9 | 150,3 | 171,7 | 171,7 | 99,68 | 98,34 | 0,65 |

Methacrolein für die DOE - Synthese kann auch in einem Batch-Verfahren gewonnen werden. Beispiel 1.21 bis 1.25. zeigt die Herstellung von Methacrolein im Batch-Prozess. Aufgrund der deutlich höheren DIMAL-Gehalte im Methacrolein kann eine Zwischenaufreinigung des Methacroleins notwendig sein.

### Beispiel 1.21 (Batchverfahren)

In einem 1 L Autoklaven werden Propionaldehyd und Formaldehyd, in Form von Formalin (in einem molaren Verhältnis von 1:1) vorgelegt. Der mittels Ölbad temperierte Autoklav wird verschlossen und mit 40 bar Stickstoff beaufschlagt. Unter Rühren wird der Inhalt auf ca. 120 °C aufgeheizt. Bei Erreichen der Solltemperatur wird die Katalysatorlösung aus Wasser, Dimethylamin und Essigsäure (0,07 Teile Dimethylamin auf ein Teil Propionaldehyd, sowie ein Verhältnis Säure zu Base von 1,1 zu 1,0) hinzugegeben. Im Feed betrug die Wasserkonzentration ca. 56 Gew%, die Beladung des Wassers mit Dimethylamin betrug 2,5 Gew%. Nach ca. 20 min wird der Versuch abgebrochen und der Autoklav in einem gerührten Eisbad gekühlt. Das Gemisch wird entnommen und mittels Phasentrennung in eine organische und eine wässrige Phase getrennt. Beide Phasen werden hinsichtlich Ihrer Zusammensetzung untersucht. Der Propionaldehyd-Umsatz beträgt 99,8 Gew%, die MAL-Ausbeute beträgt 75,9 Gew% und der DIMAL-Gehalt des Methacroleins beträgt 11,26 Gew%.

### Beispiele 1.22. bis 1.24. (Batchversuche; siehe Tabelle 2)

In einem 0,45 L Autoklaven werden Propionaldehyd und Formaldehyd in Form von Formalin (molares Verhältnis von 1:1) vorgelegt. Der mittels Ölbad temperierte Autoklav wird verschlossen und mit 40 bar Stickstoff beaufschlagt. Unter Rühren wird der Inhalt auf ca. 115 °C aufgeheizt. Bei Erreichen der Solltemperatur wird die Katalysatorlösung aus Wasser, Dimethylamin und Essigsäure hinzugegeben. Nach einer gewünschten Zeit wird der Versuch abgebrochen und der Autoklav in einem gerührten Eisbad gekühlt. Das Gemisch wird entnommen und mittels Phasentrennung in eine organische und eine wässrige Phase getrennt. Beide Phasen werden hinsichtlich Ihrer Zusammensetzung untersucht. Die Ergebnisse sind in Tabelle 2 zusammengefasst.

**Tabelle 2: Beispiele 1.22. bis 1.24.**

| | PA:FO | DMA:PA | ACOH:DMA | H2O | DMA/H2O | VWZ | TÖL | X PA | SMAL | c DIMAL |
|---|---|---|---|---|---|---|---|---|---|---|
| | mol/mol | mol% | mol/mol | Gew% | Gew% | min | °C | Gew% | Gew% | Gew% |
| 1.22 | 1 | 0,065 | 1,10 | 68 | 1,4 | 34 | 115 | 99,4 | 86 | 4,8 |
| 1.23 | 1 | 0,075 | 1,10 | 68 | 1,5 | 16 | 115 | 98,8 | 90 | 2,9 |
| 1.24. | 1 | 0,075 | 1,10 | 68 | 1,5 | 2 | 115 | 98,3 | 87 | 1,7 |

### Beispiel 1.25.

In diesem Batchverfahren (Daizel Chemical Industries 1993) werden Diethanolamin als sekundäres Amin und Oxalsäuredihydrat als katalytische Säure äquimolar zu den Reaktanden verwendet. Der vergleichsweise hohe Anteil an Katalysator erlaubt es, die Reaktion bei deutlich geringeren Temperaturen bei längeren Verweilzeiten durchzuführen. Das eingesetzte Katalysatorgemisch kann als wässrige Phase wieder für die Reaktion eingesetzt werden.

In einem Rührkessel werden 6 kg Wasser, 1274 g Diethanolamin und 1528 g Oxalsäuredihydrat vorgelegt. Anschließend werden 974 g Formalin (37%ig) und 703 g Propionaldehyd hinzudosiert. Die Reaktanden und die Katalysatoren werden äquimolar eingesetzt. Das Reaktionsgemisch wird auf 58°C unter Rückfluss für eine Dauer von ca. 20 min erhitzt. Nach Abkühlen und Destillation der dabei erhaltenen Phasen wird eine organische Phase von 850 g mit einem Methacroleingehalt von ca. 91 % erhalten, was einer Ausbeute von ca. 92 % entspricht. Die von Methacrolein befreite wässrige Phase kann erneut eingesetzt werden und besitzt annährend gleiche Aktivität wie im Batch davor.

### Beispiel 1.26.

In einem Rührkessel werden 6 kg Wasser, 1261 g Piperazin und 1069 g Adipinsäure vorgelegt und auf ca. 40°C erhitzt. Anschließend werden 1177 g Formalin (37 %ig) und 849 g Propionaldehyd zudosiert. Das entspricht einem molaren Verhältnis von 1:0.5:1:1. Nach Abschluss der Reaktion wird das Gemisch destilliert und es wird 982 g einer wässrigen Phase mit einem Methacroleingehalt von 94 % gewonnen. Dies entspricht einer Ausbeute von 91 %. Der vergleichsweise hohe Anteil an Katalysator erlaubt es, die Reaktion bei deutlich geringeren Temperaturen bei längeren Verweilzeiten durchzufuhren. Das eingesetzte Katalysatorgemisch wird als wässrige Phase wieder für die Reaktion eingesetzt.

### Beispiel 2

### 2. Kontinuierliche Durchführung einer direkten oxidativen Veresterung von Methacrolein zu Methylmethacrylat ("MMA") mit einem nanopartikulären Gold-haltigen DOE Katalysators

### 2.1. Reaktor, Reaktionssystem und Auslegungsdaten des Katalysator Rückhalte Systems

### Reaktor:

Ein Rührkesselreaktor mit aufgesetztem Rührwerk wurde für die Reaktion eingesetzt. Die eingesetzten Materialien sind aus herkömmlichem Edelstahl, um den leicht korrosiven Medien standzuhalten. Der Reaktor besitzt einen Doppelmantel, der mit einem Thermostaten verbunden ist, der über sein Medium sowohl kühlen als auch heizen kann. Der Reaktordeckel ist mit einem Brüdenrohr (100 mm Nennweite) mit einem Kondensator verbunden. Der Rührkessel hatte einen inneren Durchmesser von 400 mm, die Reaktorhöhe (bis Abschluss Deckel) betrug 1500 mm. Das Rührwerk ist von oben durch den Reaktordeckel mit dem Reaktor verbunden und ist mit speziellen Rührorganen ausgestattet, die sowohl eine optimale Gasdispergierung (für das sauerstoffhaltige Gas; verwendet wurde getrocknete Pressluft) als auch eine optimale Suspendierung des partikulären Katalysators im Medium ermöglichen. Eingesetzt wird ein kommerzielles Rührsystem bestehend aus einem Primärdispergierer für große Gasmengen (Fa. Ekato, Phasejet) und zwei Rührorgane für Gas-Flüssig-Mischungen mit radialer Förderrichtung (Fa. Ekato, Kombijet), insgesamt drei Rührorganen, die auf der Rührwelle befestigt sind. Der Abstand der Rührorgane vom Boden des Reaktors beträgt 100 mm und 400 mm für das zweite Rührorgan, sowie 700 mm für das dritte Rührorgan. Die Gasleitung für die Zuführung des sauerstoffhaltigen Gases (es wird Pressluft eingesetzt) endet direkt unter dem Dispersionsorgan und stellt eine gleichmäßige Verteilung des Gases über den Reaktorquerschnitt sicher sowie eine feine Dispergierung des Oxidationsgases. Über den Reaktordeckel sind in den Reaktor Zuführleitungen für Edukte, Recyclierströme und Hilfsstoffe verbaut, derart, dass die Zuführleitungen deutlich unterhalb des Füllstands des Reaktionsmediums enden. Der Kopf des Kondensators ist über eine Leitung mit einem Vorlagebehälter verbunden, der eine methanolische Stabilisatorlösung enthält (500 ppm Tempol).

### 2.2. Katalysatorrückhaltesystem:

Der Reaktor ist mit einem Katalysatorrückhaltesystem aus mechanischen Filtrations- und Sedimentationseinrichtungen ausgestattet. Die aus den DOE Reaktoren abgeführte Reaktionslösung wird letztlich durch eine Filterkerze mit einem Rückhaltevermögen von 1 Mikrometer gefiltert und von Feinanteilen befreit, größere Partikel, die den Filter belegen werden in regulären Abständen in den Reaktor zurückgespült um einen Verlust an katalytisch aktivem Material weitgehend zu reduzieren bzw. zu eliminieren. Als Rückspülmedium wird die Reaktionslösung selbst bzw. die pH-eingestellte Reaktionslösung nach Reaktion mit basischem Medium eingesetzt.

### 2.3. Reaktionsstart und kontinuierliche Reaktion:

Der Reaktor wurde mit einer Mischung aus Methanol, Wasser und MMA, Methacrylsäure ("MAS") sowie gelöstem Na-Methacrylat befüllt. Durch die Vorlage dieses Reaktorgemischs wird ein schnelleres Erreichen der Stationär-Konzentrationen erreicht. Der Reaktor wurde mit 135 kg dieses Start Mediums befüllt, die Zusammensetzung betrug 3 Gew% MAS (hiervon liegen bei pH 7 50% als Natriumsalz vor), 35 Gew% MMA, 5 Gew% Wasser, Rest Methanol. Der Füllstand betrug ca. 85% der Füllhöhe des Reaktors. Der Reaktor wird zusätzlich mit 15kg Katalysator gefüllt, die Slurry Dichte beträgt somit 10 -11 Gew% bzgl. eingesetzter Katalysatormenge und Arbeitsvolumen der Reaktionslösung. Die Reaktionsmischung wird auf 80°C aufgeheizt. Das Rührwerk wird auf 300 Umdrehungen eingestellt. Der Reaktor wird auf einen Arbeitsdruck von 5 bar absolut eingestellt (mit Stickstoff als Startmedium). Nach Erreichen der Reaktionstemperatur wird in Stufen von 1 kg/h Luft eingespeist, die Reaktion springt unmittelbar an, erkennbar am Absinken des Methacrolein Levels im Reaktor bzw. am Anstieg der MMA Konzentration im Reaktionsgemisch. Man steigert die Luftmenge soweit in oben genannten Stufen, bis das Abgas eine Sauerstoffkonzentration von 4 Vol.-% erreicht ist (die Explosionsgrenze des DOE Abgases liegt bei knapp 8 Vol.-% O₂ Gehalt), sodass ein sicherer kontinuierlicher Betrieb gewährleistet ist. Die Abgastemperatur des DOE Abgases nach Kondensation der kondensierbaren organischen Bestandteile (Methanol, MAL, MMA, Wasser und Leichtsieder) beträgt 2°C. Mittels NIR wird die Konzentration der verschiedenen Abgasbestandteile gemessen und quantifiziert (CO₂, CO, Sauerstoff, Wasser, Propen, Stickstoff, Methanol, Aceton, Methylformiat (FOME) und andere Leichtsieder, die mit dem Abgas ausgetragen werden). Das Abgas mit einem Gesamt-Anteil flüchtiger organischer Bestandteile < 1.5 Vol.-% wird thermisch behandelt und verbrannt.

Der Ausgangsstoff Methacrolein (hergestellt aus Propanal und Formalin in einer kontinuierlichen Druckreaktion in Gegenwart katalytischer Mengen Dimethylamin/Essigsäure) wird über eine Dosierpumpe dem Reaktor kontinuierlich zugeführt. Es werden 180 mol Methacrolein in den Reaktor pro Stunde dosiert, wobei sich im stationären Zustand ein Umsatzgrad zwischen 70-72% einstellt. Über die gesamte Laufzeit des kontinuierlichen Betriebs wird ein Umsatz von etwa 125 bis 130 Mol Methacrolein ermittelt. Der Reaktor wird nach dieser Betriebsweise mit im Wesentlichen zwei Methacrolein Feed Strömen betrieben, nämlich dem Frisch Methacrolein Feed (∼ 100 Mol/h) und dem Methacrolein Recycle Feed (dem Kopfprodukt der MAL-Methanol Recyclekolonne, bzw. eine MAL-Recovery-Kolonne) (enthält weitere -40 Mol/h MAL). Die ermittelte mittlere Reaktionsselektivität beträgt 94,5 MMA bzgl. umgesetztem Methacrolein, erzeugt werden 119 bis 124 Mol MMA pro Stunde, die Katalysatorleistung beträgt also 8,16 Mol MMA pro Stunde und kg Katalysator in dieser Ausführung.

### 2.4. Herstellung von MMA mittels DOE, Reaktoraustrag und Zusammensetzung

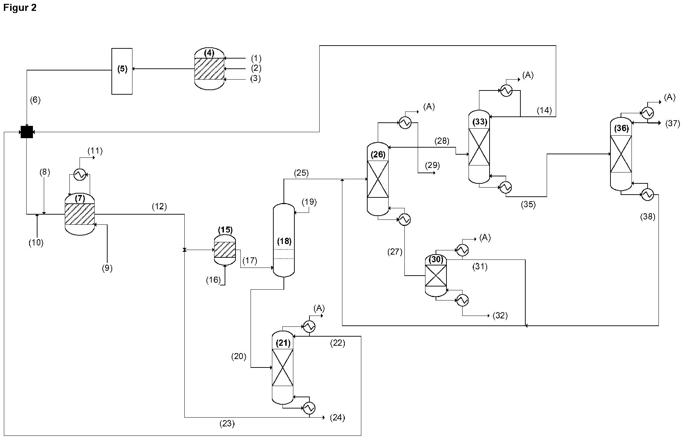

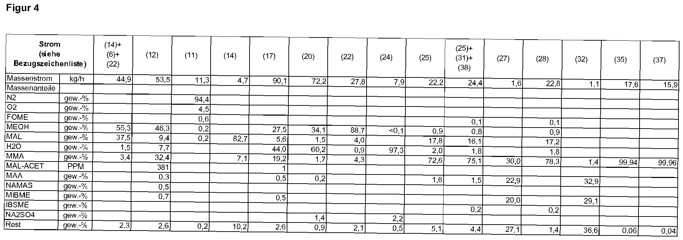

Die kontinuierliche Herstellung der Feedströme, Abgaszusammensetzung, sowie Produktströme und deren Zusammensetzung in den DOE Reaktoren (7) sind in den **Figuren 2** **und** **4** dargestellt.

Grundsätzlich sind Reaktion sowie Aufarbeitungskolonnen- und Apparate mit einem Abgassystem ausgestattet. Alle Kolonnenköpfe und kritische Apparateteile werden mit Stabilisatorlösungen kontinuierlich besprüht, um polymere Ablagerungen zu verhindern. Zusätzlich wird für delta-p Messungen abgereicherte Luft (Sauerstoffgehalt < 6 Vol%) verwendet, die am Sumpf der Kolonnen aufgegeben wird.

Der Austrag der stationären Reaktionsmischung über das Katalysatorrückhaltesystem (Schrägklärer und Filtrationseinheiten) wird über ein Druckventil gesteuert und beträgt durchschnittlich 50 bis 70 kg/h, wovon 20-30 kg in den Reaktor nach Kühlung und Basen Konditionierung recycliert werden. Der Austrag (12) zu den Aufarbeitungssektionen beträgt in dieser exemplarischen Versuchsdurchführung 45-60 kg während dieser Fahrperiode, somit ergibt sich eine durchschnittliche Reaktionszeit von etwa 4 h. Die Reaktionsmischung wird entspannt und über einen Zwischenbehälter (20 L Volumen), wobei gelöste Reste von Gas weitgehend entfernt werden, die ansonsten nachfolgende Reaktions- und Trennoperationen stören würden, geleitet. Nach Kühlung und Entspannung im Zwischenbehälter sind diese Gasbestandteile weitgehend entfernt.

Die Zusammensetzung des kontinuierlichen Reaktoraustrags wird nach der Filtrationseinheit (Polypropylenfilter Kerze mit 1 mikron Rückhaltevermögen) wird mittels NIR kontinuierlich gemessen und zusätzlich mit HPLC die Konzentration von MMA und MAS sowie nicht umgesetztes Methacrolein bestimmt und geprüft. Die niedrig konzentrierten organischen Komponenten z.B. Di-Methacrolein, Di-Methcrolein Methylester sowie Methacroleindimethylacetal (= Dimethylisobuten, MAL-ACET) werden gaschromatographisch mit Flammionisationsdetektor FID bestimmt, Wasser mittels Karl Fischer titriert und zusätzlich mit GC auf einem separaten Port bestimmt.

Die Zusammensetzung nach 24 h beträgt:

| | |
|---|---|
| MMA: | 32,4 Gew% |
| MeOH: | 46,3 Gew% |
| Methacrolein: | 9,4 Gew% |
| Methoxyisobuttersäuremethylester (MIBME): | 0,7 Gew% |
| Methacrylsäure (auch Na-MAS): | 0,8 Gew% |
| Wasser: | 7,7 Gew% |

Abgasmengen und Zusammensetzung des Abgases (11) sowie die Feedzusammensetzung (Frisch Feed und Recyclierströme) sind ebenfalls in **Figur 4** im Detail dargestellt.

Weitere vorhandene Komponenten sind Acetale des Methacroleins (MAL-ACET) mit einer Konzentration von etwa 400 ppm, sowie Isobuttersäuremethylester (IBSME), dem formalen Hydrierprodukt von MMA ebenfalls in einer Konzentration kleiner 1000 ppm in der entgasten Produktmischung, sowie Dimere des Methacroleins und deren Derivate.

Die Reaktionsproduktlösung wird mittels Zugabe von Methanol und alternativ mit Hexan (10 Teile Lösungsmittel, 1 Teil Reaktionslösung) qualitativ auf polymere Bestandteile untersucht, mit negativem Befund.

### 2.5. Brönstedtsäure Zugabe, PH Einstellung und Acetal Konvertierung, Reaktoraustrag und Zusammensetzung

Das DOE Reaktionsgemisch wird optional mit Wasser und Säure versetzt, um schädliche Acetale zu hydrolysieren. Die kontinuierliche Herstellung der Acetal-reduzierten Produktströme sowie deren Zusammensetzung nach dem MAL Acetal Konverter (15) sind **in den** **Figuren 2** **und** **4** dargestellt.

Die Mischung wird im Detail mit 96 prozentiger Schwefelsäure (16) versetzt, bis der pH der resultierenden Mischung zwischen 2 und 2,5 beträgt. Zusätzlich wird Wasser zur entgasten DOE Reaktormischung gegeben, im gewählten Ausführungsbeispiel wird Prozesswasser (23) aus der Methanol Recovery Kolonne verwendet.
Bei 40°C und 10 min Verweilzeit werden in einem gut durchmischten System (eingesetzt wird ein 5 I gerührter Reaktor mit aufgesetztem Rührelement) die vorhandenen Acetale (zum Beispiel das Methacrolein Dimethylacetal) hydrolytisch gespalten. In diesem Schritt erreicht man somit die Reduktion des Acetal Gehalts von knapp 400 ppm auf 1 ppm und somit ein unschädliches Niveau für die MMA Monomer Qualität bzw. die Qualität der PMMA Polymeren.
Es resultiert ein Gemisch (in diesem Fall zweiphasig, (17)) nach MAL Acetal Konvertierung mit folgender Zusammensetzung:

| | |
|---|---|
| MMA: | 19,2 Gew% |
| MeOH: | 27,5 Gew% |
| Methacrolein: | 5,6 Gew% |
| Methoxyisobuttersäuremethylester (MIBME): | 0,5 Gew% |
| Methacrylsäure: | 0,5 Gew% |
| Wasser: | 44,0 Gew% |
| MAL-Acetal: | 1 ppm |

Weitere vorhandene Komponenten sind Dimere des Methacroleins sowie deren Ester und deren Derivate (Hochsieder im Vergleich zu MMA), sowie Isobuttersäuremethylester (IBSME), dem formalen Hydrierprodukt von MMA.

Dieses Produktgemisch wird einer Extraktion (18) unterzogen. Die Extraktion wurde im gewählten Beispiel in einer gepulsten Extraktionskolonne mit externem Pulsationsantrieb mit einem Durchmesser von 120 mm und einer Höhe von 2500 mm betrieben. Im oberen Teil der Kolonne wird 10 bis 15 kg VE Wasser (19) dosiert, Aufgabestelle ist oberhalb der Aufgabestelle der organischen Feedlösung, 200 mm (gemessen vom Boden der Extraktionskolonne). Ziel der Operation ist die Erzeugung einer MMA, Methacrolein und Methacrylsäure-haltigen organischen Phase, mit möglichst wenig MeOH und Wasser und der Erzeugung einer wässrigen Phase, die weitgehend das entstehende Mineralsalz (im Beispiel Natriumsulfat) enthält. Nach unseren theoretischen Berechnungen bildet das gewählte apparative Set Up 4-5 theoretische Extraktionsstufen ab. Die Extraktion wird bei 40°C und Normaldruck betrieben. Im oberen Teil der Kolonne bildet sich eine klare Phasengrenze ab, die mehr oder weniger auch im Betrieb über mehrere hundert Stunden Mulch-frei verbleibt. Auch polymere Ablagerungen werden nicht beobachtet.

Die organische Phase ("zweite Phase" (25)), die kontinuierlich der folgenden Aufarbeitung zugeführt wird weist im stationären Betrieb folgende Zusammensetzung auf:

| | |
|---|---|
| MMA: | 72,6 Gew% |
| MeOH: | 0,9 Gew% |
| Methacrolein: | 17,8 Gew% |
| Methacrylsäure: | 1,6 Gew% |
| Wasser: | 2,0 Gew% |
| Andere organische Komponenten: | 5,1 Gew% |

Die organischen Komponenten in der organischen Phase bestehen aus einer Mischung von Dimeren des Methacroleins und deren Derivaten sowie Methoxyisobuttersäuremethylester (MIBME). Nach Analyse mittels Ionenchromatographie ist die organische Phase auch weitestgehend frei von Natriumsulfat (< 200 ppm).

Die wässrige Phase ("Erste Phase" (20)), die kontinuierlich der folgenden Aufarbeitung zugeführt wird weist im stationären Betrieb folgende Zusammensetzung auf:

| | |
|---|---|
| MMA: | 1,7 Gew% |
| MeOH: | 34,1 Gew% |
| Methacrolein: | 1,5 Gew% |
| Methacrylsäure: | 0,2 Gew% |
| Wasser: | 60,2 Gew% |
| Natriumsulfat: | 1,4 Gew% |

### 2.6. Aufarbeitung der "ersten Phase", Rückgewinnung von MMA, MAL, MeOH aus der wässrigen Phase

Die wässrige "erste Phase" (20) wird von Edukten und Produkten (MeOH, MAL, MMA) destillativ gereinigt. Die kontinuierliche Destillation und Rückführung organischer Wertstoffe sowie das entstehende Abwasser sind in den Figuren 2 und 4 dargestellt.

In dieser sogenannten Methanol Recovery Kolonne (Betriebsdruck 1 bar absolut (21)) wird das Methanol, sowie Reste von MMA und Methacrolein nach Extraktion zurückgewonnen. Diese Edukte und Teile des MMA werden in den Reaktor II (7) zurückgeführt und gelangen so in den organischen Aufarbeitungskreislauf.
Diese Operation erzeugt auch am Sumpf der Destillationskolonne ein Prozessabwasser (24), das mehr oder weniger vollständig das Natriumsulfat der Neutralisation enthält. Die Destillationskolonne ist mit Hochleistungspackungen DX der Firma Sulzer ausgestattet (Durchmesser 120 mm, Höhe 2000 mm) und wird bei einem Rücklaufverhältnis von 1,1 bei Normaldruck betrieben. Unter diesen Bedingungen stellt sich im Kopf der Kolonne eine Temperatur von etwa 65°C und eine Sumpftemperatur von 104°C ein.
Im Sumpf fällt Prozessabwasser (24) mit 2-2,5 Gew% Natriumsulfat an neben 97,3 Gew% Wasser, am Kopf entsteht ein Brüdenstrom (22) mit folgender Zusammensetzung:

| | |
|---|---|
| MMA: | 4,3 Gew% |
| MeOH: | 88,7 Gew% |
| Methacrolein: | 4,0 Gew% |
| Wasser: | 0,9 Gew% |

Der Sumpfstrom wird der Entsorgung (24) zugeführt bzw. ein Teilstrom (23) kann an verschiedenen Stellen des Verfahrens zurückgeführt werden. Dieser Natriumsulfat-Wasser Strom (23) wird in der vorliegenden experimentellen Beschreibung anteilig zur Unterstützung der Extraktion bzw. in den MAL-Acetal Konverter zurückgeführt, siehe Figur 2.

### 2.7. Hochsiederkolonne: Aufarbeitung der organischen "zweiten Phase", Abtrennung von MMA, MAL und Leichtsiedern von hochsiedenden Komponenten

Die organische "zweite Phase" (25) wird einer destillativen Trennung (Leichtsieder, MAL, MMA) von im Vergleich zu MMA höhersiedenden Komponenten (MAS, Di-MAL und Derivate, MIBME, etc.) unterworfen.
Die kontinuierliche Destillation und Vorreinigung zur Erzeugung von Roh-MMA sowie der entstehende organische Schwersiederstrom sind in den Figuren 2 und 4 dargestellt.

In dieser sogenannten Hochsiederkolonne (26) (Betriebsdruck 0,2 bis 0,24 bar absolut) wird das Methanol, sowie Reste von Wasser und mehr oder weniger vollständig Methacrolein zusammen mit der Hauptmenge MMA als Kopfkondensat (28) gewonnen. Das Kopfkondensat zerfällt bei gegebener Zusammensetzung in dieser experimentellen Ausführungsvariante in zwei Phasen (28) und (29).

Die organische Phase (28) enthält vor allem MMA und Methacrolein sowie geringe Mengen Wasser und Nebenprodukte:

| | |
|---|---|
| MMA: | 78,3 Gew% |
| MeOH: | 0,9 Gew% |
| Methacrolein: | 17,2 Gew% |
| Wasser: | 1,8 Gew% |

Als Nebenprodukte sind in geringen Mengen noch Methylformiat (FOME), Aceton und IBSME enthalten.

Diese Operation erzeugt auch am Sumpf der Destillationskolonne einen organischen Sumpfstrom (27), der Rückstände an MMA und vor allem höhersiedende Methacrylsäure, MIBME und Di-MAL und Derivate enthält. Auch Spuren von Mineralsalz werden in diesen Strom geschleppt und somit vom Roh-MMA getrennt. Diese organischen Nebenprodukte können weiter ausgearbeitet werden bzw. Wertstoffe wie MAS isoliert werden bzw. in speziellen Verfahren in MMA umgewandelt werden (nicht Teil der vorliegenden Erfindung).

Die Destillationskolonne ist mit Hochleistungspackungen DX, der Firma Sulzer ausgestattet (Durchmesser 100 mm, Höhe 2000 mm) und wird bei einem Rücklaufverhältnis von 1,0 bei moderatem Unterdruck betrieben. Unter diesen Bedingungen stellt sich im Kopf der Kolonne eine Temperatur von etwa 50°C und eine Sumpftemperatur von 85°C ein.

### 2.8. Aufarbeitung der organischen Schwersiederphase, finale Abtrennung von Rest MMA, von hochsiedenden Komponenten: MMA Recovery Kolonne

Die organische Schwersiederphase wird einer optionalen weiteren destillativen Trennung (30) von im Vergleich zu MMA höhersiedenden Komponenten (MAS, Di-MAL und Derivate, MIBME, etc.) unterworfen.
Der bei der kontinuierlichen Destillation bei reduziertem Druck (Betriebsdruck 0,03 bis 0,06 bar absolut, hier 0,05 bar) entstehende organische Schwersiederstrom (32) ist in den Figuren 2 und 4 dargestellt.

Grundsätzlich sind die destillativen Aufgaben der Operationen 2.7. und 2.8 in einer einzigen Kolonne bei angepassten Druck- und Temperaturparametern durchführbar. Das Destillat (31) wird als Co-Feed vor die Hochsiederkolonne Figur 2 recycliert.

### 2.9. Leichtsiederkolonne: Aufarbeitung der organischen Destillat-Phase der Schwersiederkolonne, destillative Abtrennung von Methacrolein (im Destillat) von MMA (Sumpfprodukt)

Das organische Destillat der Schwersiederkolonne (28) wird einer weiteren destillativen Trennung (33) von im Vergleich zu MMA leichtersiedenden Komponenten unterworfen. Grundsätzlich fällt ein MMA hoher Reinheit bereits hier im Sumpf (35) der Kolonne an, allerdings enthält das MMA nach dieser Variante trotz hoher Reinheit > 99 Gew% noch einige Stabilisatoren aus den vorherigen Prozessstufen.
Im Kopf ((34) oder (14)) der Kolonne fallen insbesondere gegenüber MMA leichtsiedende Stoffe an bzw. auch Komponenten, die zusammen mit MMA bzw. MAL leichtsiedende Azeotrope bilden (binäre und tertiäre Azeotrope). Die grundsätzliche Funktion der Kolonne ist die Abtrennung von Methacrolein von MMA, um eine Rückführung des enthaltenen Methacroleins in die DOE Reaktion zu erlauben.
Optional kann am Kopf der Kolonne eine Phasentrennung vorgenommen werden, um die in die DOE Reaktion rückgeführte Wassermenge zu reduzieren bzw. eine Reduktion und Ausschleusung unerwünschter Leichtsieder zu erreichen.

Das bei der kontinuierlichen Destillation bei reduziertem Druck von 600 mbar entstehende organische Leichtsieder Destillat (14) ist in den Figuren 2 und 4 dargestellt. Unter diesen Bedingungen ergibt sich im Kopf eine Temperatur von etwa 53 °C und im Sumpf von 84 °C. Der Kolonnendurchmesser beträgt 80 mm, eingebaut sind Sulzer DX Packungen (Hochleistungspackungen), Packungshöhe 2000 mm.

Das MAL reiche Destillat (14) wird als Co-Feed in die DOE Reaktorsektion (7), Figur 2, zurückgeführt.

### 2.10 MMA Reinkolonne: Aufarbeitung der organischen Sumpfphase der Leichtsiederkolonne, destillative Abtrennung von MonomerGrade MMA (im Destillat) von Roh-MMA mit hohen Stabilisatorgehalten

Der organische Sumpf der Leichtsiederkolonne (35) wird einer weiteren destillativen Trennung (36) und Feinreinigung unterworfen. Grundsätzlich fällt ein MMA entsprechend Monomer Spezifikation im Destillat (37) der Kolonne an. Im Sumpf der Kolonne fällt ein Roh-MMA mit hohem Stabilisatorgehalt (38) an.
Dieses hochstabilisierte MMA kann auf eine oder mehrere der vorherigen Kolonnen recycliert werden (dargestellt ist exemplarisch die Rückführung auf die Hochsiederkolonne (38), Figur 2). Alternativ kann dieser Strom als Stabilisatorlösung zur Aufgabe auf die kritischen Kolonnenköpfe diverser Trennoperationen verwendet werden.
Die Kolonne wird mit einem moderaten Vakuum von 220 mbar betrieben, es stellt sich eine Kopf- oder Destillattemperatur von knapp 57°C ein, bei einer Sumpftemperatur von 59°C. Der Kolonnendurchmesser beträgt 80 mm, eingebaut sind Sulzer DX Packungen (Hochleistungspackungen), Packungshöhe 2000 mm. Das Rücklaufverhältnis beträgt 1,0.

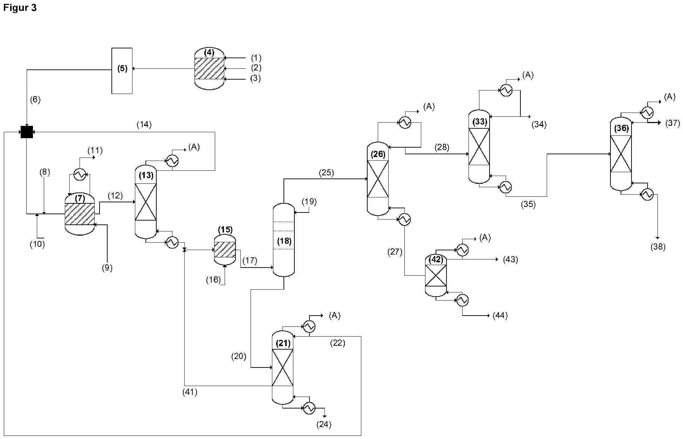

### Beispiel 3 (Vergleichsbeispiel nach Stand der Technik, Figur 3):

Vergleichsbeispiel: Dargestellt ist eine bevorzugte Aufarbeitungssequenz gemäß US 10,301,251 B2, die kurz skizziert werden soll. Details und Ablauf der verschiedenen Trennoperation sind in Figur 3 dargestellt.

Dieses Vergleichsbeispiel stellt den Stand der Technik dar und wird herangezogen, um die Vorteile des erfindungsgemäßen Verfahrens bezüglich der spezifischen Energieverbräuche, hier insbesondere Dampf- und Kühlwassereinsatz zu demonstrieren.

Methacrolein Herstellung ausgehend von Formalin und Propionaldehyd, sowie dessen Aufarbeitung, sind prinzipiell gleich ((1), (2), (3), (4), (5)). Die DOE Reaktion ((7), (8), (9), (10)) und Reaktionsführung an sich, sind ebenfalls ähnlich, unterscheiden sich allerdings in der Feedzusammensetzung, insbesondere durch unterschiedliche Recyclingströme, z.B. (14), mit unterschiedlicher Zusammensetzung.

MAL-Recovery Kolonne (13): Der DOE Reaktionsaustrag (12), bestehend aus Methanol, nicht umgesetzten Methacrolein, MMA, Wasser und Alkalisalz (oder Erdalkalisalz) der Methacrylsäure wird in einer ersten Destillationskolonne (13) aufgearbeitet. In dieser ersten Destillation wird mehr oder weniger vollständig Methacrolein, und Teile an Methanol, MMA und Wasser am Kolonnenkopf (14) abgezogen und in den DOE Reaktor (7) zurückgegeben. Der Sumpf der Kolonne enthält MMA, Wasser und Methanol sowie Methacrylsäure und Methacrylsäuresalz sowie hochsiendende Nebenprodukte.

MAL-Acetal Konvertierung: Unter Zugabe von Säure (16) (in diesem Beispiel Schwefelsäure) wird der Sumpfstrom der ersten Kolonne der Acetal Konvertierung unterzogen, bei einem pH Wert deutlich unterhalb des pHs der Reaktionslösung im DOE Reaktor. In der Acetal Konvertierung werden Spuren des störenden Acetals fast vollständig hydrolisiert, optional wird Wasser zugegeben. Gemäß US 10,301,251 B2 wird beispielsweise eine zusätzliche Wasserzugabe optional über einen wasserhaltigen Brüden Seitenstrom (41) aus der Methanol Recovery Kolonne (21) realisiert. Dies hat den Vorteil, dass neben Wasser auch Reste von Methacrylsäure (die im Abwasser sind) gleichzeitig wieder der Aufarbeitung zugeführt werden und so nicht verloren gehen. Das Produkt (17) aus dem Acetal Konverter wird einer mehrstufigen Extraktion (18) zugeführt (in Figur 3 simplifiziert als eine kontinuierliche Extraktionskolonne dargestellt).

Extraktion und MEOH Recovery Kolonne: Die wässrige Phase (20) der Extraktion enthält zu großen Teilen Methanol und geringe Mengen an MMA. In der MEOH Recovery Kolonne werden diese Wertstoffe (MEOH und MMA) als Kopfprodukt (22) zurückgewonnen und in die Reaktion (7) oder Aufarbeitung zurückgeführt. Optional wird ein wässriger Seitenstrom (41) abgezogen, der geringe Mengen an Methacrylsäure und größere Mengen Wasser enthält. Im Sumpf (24) fällt das Abwasser der DOE an, mit geringen Mengen an organischen Verunreinigungen und mehr oder weniger MMA, MAS und Methanol frei. Der Abwasserstrom enthält das Neutralisationssalz, im gewählten Beispiel Natriumsulfat.

Extraktion und organische Phase der Extraktion: die organische Phase (25) der Extraktion besteht im wesentlichen aus MMA mit geringen Mengen an Methanol, Wasser und Methacrolein (übrigens ein zentrales unterschiedliches Merkmal zum vorliegenden erfindungsgemäßen Verfahren). Auch Spuren störender Leichtsieder sind hier enthalten (z.B. Methylformiat), die entfernt werden müssen, sowie organische hochsiedende Produkte wie dimere Methacrolein und seine Derivate (z.B. Di-MAL Methylester und MIBME). MMA wird in einer Sequenz von Destillationskolonnen so aufgereinigt, damit die Spezifikation für Monomer Grade erreicht werden kann. Hochsiederkolonne (26): Hierzu wird in einer Hochsiederkolonne (26) zunächst alle höhersiedenden Komponenten abgetrennt. MMA geht am Kopf (28) dieser Kolonne zusammen mit Leichtsiedern bzw. als Azeotrop mit diesen Leichtsiedern. Methacrylsäure kann aus dem Sumpfstrom (27) in einer optionalen Fraktionierung (dargestellt in Figur 3, sog. "MAS Kolonne" (42)) von anderen Hochsiedern abgetrennt und isoliert werden.
Leichtsiederkolonne (33): In der Leichtsiederkolonne wird das Destillat der Hochsiederkolonne aufgearbeitet; alle leichtsiedenden Komponenten werden von Roh-MMA (35) abgetrennt, das im Sumpf zusammen mit den Prozessstabilisatoren anfällt.
MMA Reinkolonne (36): In der MMA Reinkolonne (36) werden die Stabilisatoren von spezifikationsgerechtem MMA (als Destillat (37)) abgetrennt. Dieser Sumpfstrom (38) kann optional recycliert werden, um MMA Verluste zu minimieren.
In Tabelle 3 werden die mittels Simulation ermittelten spezifischen Energieverbräuche der Verfahrensvariante (Figur 3, US 10,301,251 B2) mit denen des erfindungsgemäßen Verfahrens (Figur 2) verglichen.

### Tabelle 3:

Vergleich der spezifischen Energieverbräuche bei vorgegebener gleicher Produktreinheit (MMA Spezifikation).
a. Aufarbeitungssequenz gemäß gemäß Beispiel 3 (Figur 3)
b. Aufarbeitungssequenz gemäß erfindungsgemäßem Verfahren (Figur 2).

| Energien und Ströme zur Entsorgung | Einheit | Beispiel 3 Vergleichsbeispiel | Beispiel 2 Erfindungsgemäßes Verfahren |
|---|---|---|---|
| Dampf | kg_{Dampf}/kg_{MMA} | 4,95 | 4,25 |
| Kühlwasser | I_{Kühlwasser}/kg_{MMA} | 355 | 294 |
| Sole | kW_{Kühlleistung}/kg_{MMA} | 0,19 | 0,17 |
| Organischer Abfall | kg_{Abfall}/kg_{MMA} | 0,08 | 0,07 |
| Abwasser | kg_{Abwasser}/kg_{MMA} | 0,61 | 0,50 |

### Figuren- und Bezugszeichenliste

Folgende Figuren finden sich im Anhang:
Figur 1:
   Gesamtverfahren Ausführungsvariante: PA + FA → MAL ; 2.) MAL + O2 + MeOH → MMA + H2O, sowie eine exemplarische Aufarbeitungssequenz, hier inklusive separater Methylformiatkolonne
Figur 2:
   Gesamtverfahren Ausführung: PA + FA → MAL ; 2.) MAL + O2 + MeOH → MMA + H2O, sowie eine exemplarische Aufarbeitungssequenz, hier ohne separate Methylformiatkolonne
Figur 3:
   Vergleichsprozess nach Stand der Technik gemäß US 10,301,251 B2 (Groemping et al.) Gesamtverfahren Ausführung: PA + FA → MAL ; 2.) MAL + O2 + MeOH → MMA + H2O, sowie eine exemplarische Aufarbeitungssequenz, mit extraktiver Aufarbeitung einer Methacrylsäure Alkalisalz enthaltenden MMA-Methanol Mischung
Figur 4:
   Massenströme und Zusammensetzung nach Beispiel 2 und Figur 2

### Bezugszeichen:

### Oxidative Veresterung des Methacroleins zu einem Alkylmethacrylat und Recyclierung des Methacroleins

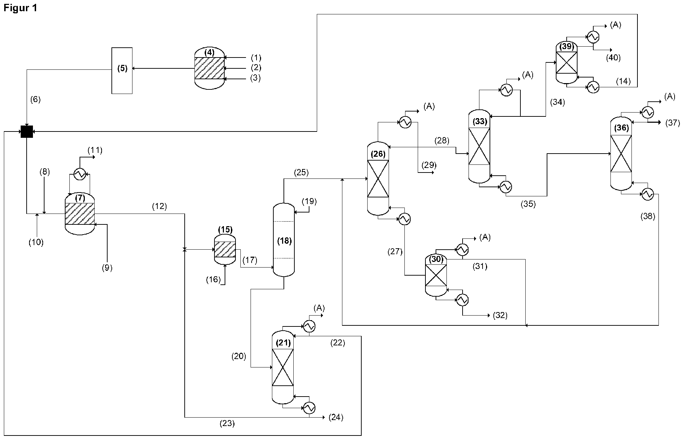

(1) Formalin-Zulauf in Reaktor I
(2) Propanal-Zulauf in Reaktor I
(3) Optionaler Stabilisator-Zulauf in Reaktor I
(4) Reaktor I zur Methacroleinsynthese
(5) Aufbereitung des Roh-Methacroleins
(6) Methacrolein-Zulauf in Reaktor II
(7) Reaktor II zur oxidativen Veresterung des Methacroleins
(8) Alkohol-Zulauf (i.d.R. Methanol-Zulauf)
(9) Sauerstoff- bzw. Luftzuleitung
(10) Zulauf Base
(11) Abgas Reaktor II
(12) Reaktoraustrag Reaktor II
(13) Destillationskolonne MAL-Recovery Kolonne (nur Vergleichsbeispiel Figur 3)
(14) MAL Recycle
(15) Optionaler Mischer / MAL-Acetal-Converter
(16) Zulauf Säure (i.d.R. Schwefelsäure)
(17) Produktstrom MAL-Acetal-Converter
(18) Extraktion
(19) Wasser-Zulauf für Extraktion
(20) Wässrige Phase Extraktion "erste Phase"
(21) Methanol-Recovery Kolonne
(22) Niedrigsiedende Fraktion, enthaltend Alkohol zur Rückführung in Reaktor II
(23) Optionale Sumpf-Fraktion Rückführung der Methanol-Recovery Kolonne
(24) Sumpf-Fraktion, enthaltend Wasser, Säure und deren Alkalisalze, zur Entsorgung oder weiteren Aufarbeitung,
(25) Organische Phase der Extraktion "zweite Phase"
(26) Hochsiederkolonne zur Abtrennung von Hochsiedern
(27) Sumpf-Fraktion ("dritte Phase"), enthaltend MMA, Methacrylsäure und Schwersieder
(28) Destillat ("vierte Phase"), enthaltend MMA und Leichtsieder
(29) Wässrige Phase Destillat der Hochsiederkolonne
(30) MMA-Recovery Kolonne
(31) Destillat der MMA-Recovery Kolonne, enthaltend MMA, zur Rückführung in die Hochsiederkolonne
(32) Sumpf-Fraktion der MMA-Recovery Kolonne, enthaltend Methacrylsäure und Schwersieder
(33) Leichtsiederkolonne zur Abtrennung von Leichtsiedern
(34) Destillat der Leichtsiederkolonne, enthalten Leichtsieder (Variante 1 (Figur 1) und Vergleichsbeispiel (Figur 3))
(35) Sumpf-Fraktion der Leichtsiederkolonne, enthaltend von Leichtsiedern gereinigtes MMA
(36) MMA Reinkolonne zur Endreinigung des MMA
(37) Spezifikationsgerechtes MMA als Destillat der MMA Reinkolonne
(38) Sumpf-Fraktion der MMA Reinkolonne optionale Rückführung zur Hochsiederkolonne
(39) Methylformiat Kolonne (nur Variante 1 Figur 1)
(40) Destillat Methylformiat Kolonne enthält Leichtsieder, wie beispielsweise Methylformiat
(41) Optionaler Seitenstrom Methanol Recovery Kolonne (nur Vergleichsbeispiel Figur 3)
(42) MAS Kolonne (nur Vergleichsbeispiel Figur 3)
(43) Destillat MAS Kolonne, enthält Methacrylsäure (nur Vergleichsbeispiel Figur 3)
(44) Sumpf-Fraktion der MAS Kolonne, enthält Schwersieder (nur Vergleichsbeispiel Figur 3)
(A) Abgas

## Patentansprüche

1. Verfahren zur Herstellung von Alkylmethacrylaten, bei dem in einer ersten Reaktionsstufe in einem Reaktor I Methacrolein hergestellt und dieses in einer zweiten Reaktionsstufe in einem Reaktor II oxidativ mit einem Alkohol in flüssiger Phase zu einem Alkylmethacrylat verestert wird, **dadurch gekennzeichnet, dass** der Reaktoraustrag aus Reaktor II in eine erste wässrige Phase, enthaltend mehr als 80 Gew% des im Reaktoraustrag vorliegenden Alkohols und eine zweite Phase, enthaltend jeweils mehr als 80 Gew% des im Reaktoraustrag vorliegenden Alkylmethacrylats und Methacroleins (MAL) getrennt wird und in einer nachfolgenden ein- oder mehrstufigen Destillation die zweite Phase so aufgearbeitet wird, dass Methacrolein von Alkylmethacrylat getrennt wird.

2. Verfahren gemäß Anspruch 1 zur Herstellung von Alkylmethacrylaten, bei dem in einer ersten Reaktionsstufe in einem Reaktor I Methacrolein hergestellt und dieses in einer zweiten Reaktionsstufe in einem Reaktor II oxidativ mit einem Alkohol zu einem Alkylmethacrylat verestert wird, **dadurch gekennzeichnet, dass** der Reaktoraustrag aus Reaktor II in eine erste Phase, enthaltend mehr als 80 Gew% des im Reaktoraustrag vorliegenden Alkohols und eine zweite Phase, enthaltend jeweils mehr als 90 Gew% des im Reaktoraustrag vorliegenden Alkylmethacrylats und Methacroleins (MAL) getrennt wird, und dass die zweite Phase derart aufgearbeitet wird, dass
a. destillativ zuerst eine Abtrennung von Alkylmethacrylat und Methacrolein von schwersiedenden Komponenten erfolgt und anschließend die destillative Abtrennung von Methacrolein von relativ höher siedendem Alkylmethacrylat oder
b. destillativ zuerst eine Abtrennung von Alkylmethacrylat und schwersiedenden Komponenten von Methacrolein erfolgt und anschließend die destillative Abtrennung von Alkylmethacrylat von relativ hierzu schwersiedenden Komponenten
bevor Alkylmethacrylat in einer letzten Destillation als Kopfprodukt in Monomerqualität erhalten wird.

3. Verfahren gemäß einem der vorigen Ansprüche, **dadurch gekennzeichnet, dass** es sich bei dem Alkohol um Methanol und bei dem Alkylmethacrylat um MMA handelt.

4. Verfahren gemäß Anspruch 1 oder 2, **dadurch gekennzeichnet, dass** die vierte Phase erst in einem Phasentrenner von mindestens 90 Gew% des in der vierten Phase vorliegenden Wassers getrennt wird, bevor in einer Destillationskolonne der überwiegende Teil des Alkylmethacrylats vom überwiegenden Teil des MAL getrennt wird.

5. Verfahren gemäß mindestens einem der Ansprüche 1 bis 3, **dadurch gekennzeichnet, dass** die vierte Phase oder die organische Phase der vierten Phase nach einer Trennung von einer wässrigen Phase in einem Phasentrenner in einer Destillationskolonne in eine leichtsiedende Fraktion, enthaltend mindestens 60 Gew% des MAL des Reaktoraustrags und eine schwersiedende Phase, enthaltend mindestens 90 Gew% des MMA des Reaktoraustrags getrennt wird.

6. Verfahren gemäß Anspruch 4, **dadurch gekennzeichnet, dass** die leichtsiedende, MAL enthaltende Fraktion in einer weiteren Kolonne von leichtsiedenden Bestandteilen getrennt wird und eine schwersiedende Fraktion dieser Kolonne, enthaltend mindestens 60 Gew% des MAL des Reaktoraustrags in den Reaktor II oder einen dem Reaktor II vorgeschalteten Mischer oder Wärmetauscher geleitet wird.

7. Verfahren gemäß Anspruch 5, **dadurch gekennzeichnet, dass** die MAL enthaltene Fraktion der vierten Phase erst in einem Phasentrenner von einer wässrigen Phase getrennt wird, bevor die destillative Trennung von leichtsiedenden Bestandteilen und die Weiterleitung der MAL-Fraktion in oder vor Reaktor II erfolgt.

8. Verfahren gemäß mindestens einem der Ansprüche 1 bis 6, **dadurch gekennzeichnet, dass** der Reaktoraustrag aus Reaktor II erst in einem Konverter mit einer starken Säure und optional zusätzlichem Wasser versetzt wird, bevor eine extraktive Trennung der ersten von der zweiten Phase erfolgt, und dass die erste Phase in einer weiteren Destillationskolonne in eine überwiegend Alkohol enthaltene Phase und eine schwersiedende Phase, die entsorgt wird, getrennt wird.

9. Verfahren gemäß Anspruch 8, **dadurch gekennzeichnet, dass** so viel der starken Säure zugegeben wird, dass der pH-Wert bei der Extraktion immer ≥ 1 und kleiner gleich 5 ist.
